# FASCICULE DE BREVET EUROPEEN

(11) **EP 2 350 315 B1**
(45) Date de publication et mention de la délivrance du brevet: **31.07.2013**
(21) Numéro de dépôt: 09759748.8
(22) Date de dépôt: 21.10.2009
(51) Int. Cl.: C12Q 1/68

(54) **PROCÉDÉ DE DÉTECTION DE LA MALADIE ATHÉROMATEUSE**
VERFAHREN ZUM NACHWEIS VON ATHEROMATÖSER KRANKHEIT
METHOD FOR DETECTING ATHEROMATOUS DISEASE

(30) Priorité: 21.10.2008 FR 0805827
(43) Date de publication de la demande: 03.08.2011
(73) Titulaire: COHIRO, 21000 Dijon (FR)
(72) Inventeur: GIROUX, Sébastien, F-71290 Cuisery (FR); PESANT, Matthieu, F-21000 Dijon (FR); CONNAT, Jean-Louis, F-21490 Varois et Chaignot (FR); ROCHETTE, Luc, F-21490 Brognon (FR)
(74) Mandataire: Oudin, Stéphane
(86) Numéro de dépôt international: PCT/FR2009/001232
(87) Numéro de publication internationale: WO 2010/046564

(56) Documents cités:
- WO-A-2007/121495
- MCCALL SAMUEL H ET AL: "Osteoblasts express NLRP3, a nucleotide-binding domain and leucine-rich repeat region containing receptor implicated in bacterially induced cell death." JOURNAL OF BONE AND MINERAL RESEARCH : THE OFFICIAL JOURNAL OF THE AMERICAN SOCIETY FOR BONE AND MINERAL RESEARCH JAN 2008, vol. 23, no. 1, janvier 2008 (2008-01), pages 30-40, XP002526384 ISSN: 0884-0431
- LI S-H ET AL: "INTRANUCLEAR HUNTINGTIN INCREASES THE EXPRESSION OF CASPASE-1 AND INDUCES APOPTOSIS" HUMAN MOLECULAR GENETICS, OXFORD UNIVERSITY PRESS, SURREY, vol. 9, no. 19, 22 novembre 2000 (2000-11-22), pages 2859-2867, XP000990496 ISSN: 0964-6906
- GENG YONG-JIAN ET AL: "Evidence for apoptosis in advanced human atheroma: Colocalization with interleukin-1-beta-converting enzyme" AMERICAN JOURNAL OF PATHOLOGY, vol. 147, no. 2, 1995, pages 251-266, XP008105946 ISSN: 0002-9440
- YOUNG J L ET AL: "THE SERPIN PROTEINASE INHIBITOR 9 IS AN ENDOGENOUS INHIBITOR OF INTERLEUKIN 1BETA-CONVERTING ENZYME (CASPASE-1) ACTIVITY IN HUMAN VASCULAR SMOOTH MUSCLE CELLS" JOURNAL OF EXPERIMENTAL MEDICINE, ROCKEFELLER UNIVERSITY PRESS, JP, vol. 191, no. 9, 1 mai 2000 (2000-05-01), pages 1535-1544, XP000946391 ISSN: 0022-1007
- SWIRSKI FILIP K ET AL: "Ly-6Chi monocytes dominate hypercholesterolemia-associated monocytosis and give rise to macrophages in atheromata." THE JOURNAL OF CLINICAL INVESTIGATION JAN 2007, vol. 117, no. 1, janvier 2007 (2007-01), pages 195-205, XP002526385 ISSN: 0021-9738
- TACKE FRANK ET AL: "Monocyte subsets differentially employ CCR2, CCR5, and CX3CR1 to accumulate within atherosclerotic plaques." THE JOURNAL OF CLINICAL INVESTIGATION JAN 2007, vol. 117, no. 1, janvier 2007 (2007-01), pages 185-194, XP002526386 ISSN: 0021-9738
- GORDON SIAMON: "Macrophage heterogeneity and tissue lipids." THE JOURNAL OF CLINICAL INVESTIGATION JAN 2007, vol. 117, no. 1, janvier 2007 (2007-01), pages 89-93, XP002526387 ISSN: 0021-9738
- YAN ZHONG-QUN ET AL: "Innate immunity, macrophage activation, and atherosclerosis." IMMUNOLOGICAL REVIEWS OCT 2007, vol. 219, octobre 2007 (2007-10), pages 187-203, XP002526388 ISSN: 0105-2896

### LISTAGE DES SEQUENCES

<110> UNIVERSITE DE BOURGOGNE
<120> PROCEDE DE DETECTION DE LA MALADIE ATHEROMATEUSE
<130> UBG001-WO-23
<150> FR200805827
   <151> 2008-10-21
<160> 10
<170> PatentIn version 3.5
<210> 1
   <211> 20
   <212> DNA
   <213> Mus musculus
<400> 1
   gcaggaggaa gactttgtgc 20
<210> 2
   <211> 20
   <212> DNA
   <213> mus musculus
<400> 2
   aggagatgtc gaagcagcat 20
<210> 3
   <211> 20
   <212> DNA
   <213> mus musculus
<400> 3
   accctcaagt tttgcccttt 20
<210> 4
   <211> 20
   <212> DNA
   <213> mus musculus
<400> 4
   tctttcaagc ttgggcactt 20
<210> 5
   <211> 20
   <212> DNA
   <213> mus musculus
<400> 5
   ctcacaagca gagcacaagc 20
<210> 6
   <211> 20
   <212> DNA
   <213> mus musculus
<400> 6
   ctcagtgcag gctatgacca 20
<210> 7
   <211> 20
   <212> DNA
   <213> mus musculus
<400> 7
   gcctcaaacc ttccaaatca 20
<210> 8
   <211> 20
   <212> DNA
   <213> mus musculus
<400> 8
   tggatccatt tcctcaaagg 20
<210> 9
   <211> 21
   <212> DNA
   <213> mus musculus
<400> 9
   ctgaaggtca aagggaatgt g 21
<210> 10
   <211> 21
   <212> DNA
   <213> mus musculus
<400> 10
   ggacagagtc ttgatgatct c 21

## Description

La présente invention concerne le domaine des maladies cardio-vasculaires et plus spécifiquement l'athérosclérose. En particulier, la présente divulgation a pour objet de proposer des procédés et des kits permettant le diagnostic précoce de la maladie athéromateuse.

Les taux élevés de cholestérol dans le sang chez les patients, et plus particulièrement du cholestérol de type LDL, c'est-à-dire constitué par des lipoprotéines de faible densité, provoquent des accumulations de dépôts lipidiques au niveau des artères sous la forme d'athéromes qui conduisent à l'athérosclérose. Cette dernière entraîne un rétrécissement du diamètre artériel et s'accompagne donc d'un risque majeur de formation de caillots sanguins (thrombus) pouvant conduire à l'occlusion des artères concernées ou d'autres territoires vasculaires si le thrombus est transporté par la circulation sanguine.

Dans la lutte contre l'hypercholestérolémie, l'utilisation des statines (ou inhibiteurs de la HMG-CoA réductase) est bien connue. Toutefois, si ces molécules ont démontré leur efficacité dans la diminution du taux de cholestérol dans le sang et dans la diminution du risque de la survenue ou de récidive de maladies résultant du rétrécissement ou de l'occlusion d'artères, en limitant la progression de la plaque athéromateuse, elles n'ont a priori pas d'effet démontré contre l'installation desdites plaques d'athérome. De surcroît, certaines statines se sont avérées engendrer des effets secondaires suffisamment graves pour entraîner leur retrait du marché.

Il a maintenant été établi que la maladie athéromateuse est une maladie inflammatoire chronique. Un paramètre important dans l'initiation et le développement de la maladie est l'infiltration et l'oxydation du cholestérol de type LDL dans la paroi vasculaire. Le cholestérol oxydé entraîne l'activation des cellules endothéliales ce qui induit le recrutement de cellules inflammatoires (tel que les monocytes, les macrophages et les lymphocytes T) et la production d'une quantité importante de cytokines et de chemiokines. Parmi celles-ci, on peut citer notamment l'Interleukine 1β (IL-1β) et l'Interleukine 18 (IL-18). Ces deux molécules sont directement impliquées dans la maladie athéromateuse comme l'ont montré les travaux effectués avec des souris n'exprimant pas les molécules en question. Ces cytokines sont produites sous la forme de pro-cytokines inactives qui sont ensuite clivées par la Caspase-1, c'est sous cette forme clivée qu'elles deviennent matures et actives.

Il a été récemment démontré que l'activation de la Caspase-1 se faisait au coeur de complexes multiprotéiques plus larges nommés inflammasomes. Parmi ceux-ci, l'inflammasome Nlrp3 est exprimé dans un grand nombre de tissus et plus particulièrement dans le coeur et dans les cellules immunocompétentes telles que les monocytes et les macrophages. En plus de la caspase-1, l'inflammasome Nlrp3 est également composé de la protéine ASC qui lie la protéine Nlrp3 à un dimère de pro-caspase-1.

Il a été décrit, dans la demande de brevet WO2007/121495, un procédé de diagnostic de l'athérosclérose à partir d'un échantillon sanguin comprenant la mesure du niveau d'expression de la caspase-1

La présente invention est basé sur la mise en évidence que l'apparition de la maladie athéromateuse est liée à la sur-expression des composants de l'inflammasome Nlrp3 dans un type particulier de monocytes, les monocytes GR1high murins ou leurs homologues dans les autres espèces (notamment chez l'homme).

Il est connu de McCall et al. (Journal of Bone and Mineral Research, 23, 1, 2008, 30-40) que le niveau d'expression de l'ASC et du NLRP3 par les ostéoclastes est lié au processus d'apoptose des ostéoclastes et plus particulièrement au processus d'apoptose induit par le contact avec la bactérie salmonella enterica. Toutefois, McCall et al. ne mentionne pas la maladie athéromateuse, ni les cellules GR1high.

Il est également connu de Li et al. (Human Molecular Genetics, 200, 9, 19, 2859-2867) que l'expression intranucléaire de l'huntingtin (une protéine impliquée dans la maladie de Huntington) induit l'expression de la caspase-1. Toutefois, Li et al. ne mentionne pas la maladie athéromateuse, ni les cellules GR1high.

Il est également connu de Geng et al. (American Journal of Pathology, 147, 2, 251-266) que les macrophages présents dans les plaques athéromateuses présentent des marqueurs spécifiques de l'apoptose et que les plaques athéromateuses exprime l'ICE (un autre nom pour la capase-1). Toutefois, Geng et al. ne mentionne pas le rôle spécifique des cellules GR1high.

Ainsi la présente invention, concerne notamment un procédé de diagnostic in vitro de la maladie athéromateuse à partir d'un échantillon biologique d'un patient ou d'un animal remarquable en ce qu'il comprend la mesure du niveau d'expression de Nlrp3, d'ASC et/ou de caspase-1 par les cellules GR1high.

Le terme « échantillon biologique » fait référence à tous les fluides ou tissus contenant des monocytes du patient ou de l'animal. Préférentiellement, l'échantillon biologique est le sang du patient ou de l'animal.

Le procédé selon l'invention peut être mis en oeuvre avec un échantillon biologique brut (i.e. tel que prélevé sans avoir subi de modification), mais également après traitement de l'échantillon biologique par toutes les méthodes jugées souhaitables par l'homme du métier. Parmi ces traitements on peut citer la lyse des érythrocytes, l'isolement des cellules mononuclées (e.g. isolement sur Ficoll) et/ou l'ajout de molécules permettant la conservation de l'échantillon biologique (e.g. antiprotéases). Des techniques telles que la lyse des érythrocytes et l'isolement des cellules mononuclées permettent notamment d'augmenter la proportion de cellules GR1high par rapport aux autres cellules présentes dans l'échantillon biologique. Ainsi, selon un mode de réalisation préféré, le procédé de diagnostic selon l'invention comprend en outre une étape permettant d'augmenter la proportion des cellules GR1high par rapport aux autres cellules présentes dans l'échantillon biologique.

Dans la présente demande, dans le cas où l'échantillon biologique à traiter est un échantillon humain, le terme « Nlrp3 » fait référence à un ADN ayant plus de 90%, de préférence 95%, de manière encore plus préféré 99% et de façon tout à fait préféré 100% d'homologie avec la séquence ayant le numéro d'accès NM_001079821.1, NM_001127461.1, NM_001127462.1, NM_004895.3 ou NM_183395.1 dans la base de donnée GenBank, aux ARNm correspondant et aux protéines codées par lesdits ARNm. En ce qui concerne la protéine, il peut s'agir de la protéine avant ou après modifications post-traductionnelles et entière ou clivée. Le procédé concerne la détection de tout ou partie de Nlrp3, ainsi il est possible de ne détecter qu'une partie de l'ARNm ou de la protéine, pour autant que le niveau d'expression de cette partie est représentatif de l'expression de la totalité de la molécule.

Dans la présente demande, dans le cas où l'échantillon biologique à traiter est un échantillon humain, le terme « ASC » fait référence, à un ADN ayant plus de 90%, de préférence 95%, de manière encore plus préféré 99% et de façon tout à fait préféré 100% d'homologie avec la séquence ayant le numéro d'accès ABO23416 dans la base de donnée GenBank, à l'ARNm correspondant et à la protéine codée par ledit ARNm. En ce qui concerne la protéine, il peut s'agir de la protéine avant ou après modifications post-traductionnelles et entière ou clivée. Le procédé concerne la détection de tout ou partie de l'ASC, ainsi il est possible de ne détecter qu'une partie de l'ARNm ou de la protéine, pour autant que le niveau d'expression de cette partie soit représentatif de l'expression de la totalité de la molécule.

Dans la présente demande, dans le cas où l'échantillon biologique à traiter est un échantillon humain, le terme « caspase-1 » fait référence, à un ADN ayant plus de 90%, de préférence 95%, de manière encore plus préféré 99% et de façon tout à fait préféré 100% d'homologie avec la séquence ayant le numéro d'accès U13697 ou le numéro d'accès NM_033292 dans la base de donnée GenBank, à l'ARNm correspondant et à la protéine codée par ledit ARNm. En ce qui concerne la protéine, il peut s'agir de la protéine modifications post-traductionnelles et entière ou clivée,. Le procédé concerne la détection de tout ou partie de caspase-1, ainsi il est possible de ne détecter qu'une partie de l'ARNm ou de la protéine, pour autant que le niveau d'expression de cette partie soit représentatif de l'expression de la totalité de la molécule.

Dans le cas où l'échantillon biologique à traiter provient d'une autre espèce, les termes « Nlrp3 », « ASC » et « caspase-1 » correspondent aux équivalents dans ladite espèce des molécules humaines décrites ci-dessus.

Dans la présente demande, le terme « niveau d'expression » fait référence à la quantité de Nlrp3, d'ASC et/ou de caspase-1 exprimée par les cellules. La mesure du niveau d'expression peut être faite de manière quantitative ou semi quantitative, en effet il n'est pas nécessaire de connaître la quantité exacte de Nlrp3, d'ASC et/ou de caspase-1, exprimée par les cellules, mais simplement de déterminer si cette quantité est significativement supérieure à une norme. Cette norme peut être facilement déterminée par l'homme du métier en utilisant un pool d'individus sains et en mesurant le niveau d'expression de Nlrp3, d'ASC et/ou de caspase-1 dans les cellules de ces individus. Si le procédé suivant l'invention révèle un niveau d'expression de Nlrp3, d'ASC et/ou de caspase-1p10 dans les cellules des patients supérieur au niveau d'expression normal, ledit patient sera susceptible d'être atteint de la maladie athéromateuse.

Dans la présente demande le terme "cellules GR1high" fait référence aux monocytes murins, ainsi qu'a leurs homologues dans les autres espèces (notamment chez l'homme), exprimant le CCR2 et la L-selectin contrairement aux autres monocytes qui n'expriment pas ces deux molécules (également appelé GR1low).

Selon un mode de réalisation préféré, la mesure du niveau d'expression de Nlrp3, d'ASC et/ou de caspase-1 est la mesure du niveau d'expression de l'ARNm codant le Nlrp3, l'ASC ou la caspase-1. Les techniques permettant de mesurer la quantité d'ARNm codant spécifiquement une molécule sont bien connues de l'homme du métier. Parmi ces techniques ont peu citer la RT-PCR quantitative, la RT-PCR semi-quantitative, le northern blot et la technique des puces à ADN (en anglais « microarrays »). La construction et la production des sondes et des oligonucléotides nécessaires à la mise en oeuvre de ces techniques sont à la portée de l'homme du métier qui peut s'inspirer des sondes oligonucléotides décrites dans les exemples. Ainsi, selon un mode de réalisation encore plus préféré la mesure du niveau d'expression de l'ARNm codant le Nlrp3, l'ASC ou la caspase-1 est faite par RT-PCR quantitative,RT-PCR semi-quantitative, northern blot ou par la technique des puces à ADN.

Selon un autre mode de réalisation préféré, la mesure du niveau d'expression de Nlrp3, d'ASC et/ou de caspase-1 est la mesure de la quantité de la protéine Nlrp3, de la protéine ASC et/ou de la protéine caspase-1 à l'intérieur des cellules GR1high.

Dans le cadre de la présente demande, le terme « méthode immunologique » fait référence aux techniques de détection des protéines faisant appel à des anticorps spécifiques de ladite protéine. Dans le cadre de la présente demande, le terme « anticorps » fait référence à un polypeptide comprenant au moins un paratope. Parmi les anticorps, on peut citer les récepteurs des cellules T, les immunoglobulines, les anticorps chimériques, les anticorps humains, les anticorps humanisés, les anticorps monoclonaux, les anticorps recombinants et les fragments d'anticorps. Parmi les fragments d'anticorps, on peut citer les Fab, Fab', F(ab)2, F(ab')2, Fv et les scFv.

Ainsi selon un mode de réalisation encore plus préféré, le procédé de diagnostic selon l'invention comprend en outre une étape où la protéine Nlrp3, l'ASC et/ou la caspase-1p10 est mise en contact avec un anticorps spécifique de ladite protéine.

Les techniques permettant de mesurer le niveau d'expression d'une protéine intracellulaire sont bien connues de l'homme du métier. On peut citer notamment l'ELISA, la cytométrie de flux, le Western-Blot. Ainsi selon un mode de réalisation encore plus préféré, la méthode immunologique est choisie parmi le groupe comprenant l'ELISA, la Cytométrie de flux, le Western-blot

Les méthodes immunologiques peuvent être notamment employées sur des lysats cellulaires ou sur des cellules dont la membrane est perméabilisée afin de permettre le passage des anticorps à l'intérieur de la cellule.

Finalement, la présente divulgation concerne également des kits permettant de mettre en oeuvre le procédé suivant l'invention. Selon un mode de réalisation préféré, le kit selon l'invention comprend au moins un anticorps spécifique de Nlrp3, de l'ASC et/ou de caspase-1.

Selon un autre mode de réalisation préféré, le kit de diagnostic comprend au moins une sonde oligonuclétodique spécifique de l'ARNm codant Nlrp3, l'ASC et/ou de caspase-1.

### Matériels et méthodes :

Toutes les procédures décrites ci-dessous sont conformes au « Guide for the Care and Use of Laboratory Animals" publié par le NIH (NIH Publication No. 85-23, révision 1996).

### Animaux :

Des souris males LDLR-/- C57BL/6 de 13 semaines ont été utilisées (Jackson Laboratory). Pour l'analyse de l'expression des composants de l'inflammasome Nlrp3 dans les plaques athéromateuses, les souris (4 par groupes) ont été nourries avec un régime riche en cholestérol (1.25% de cholestérol, 0% de cholate, Research Diet, ref D12108) pendant 6 semaines. Pour le prélèvement, les souris ont été anesthésiées par de l'isofluran et saignées par piqûre dans la veine cave.

### Analyse des lésions athéromateuses par Immunohistochimie :

Après anesthésie, le coeur et l'aorte des souris ont été perfusés avec du PBS (tampon phosphate) contenant 4% de PAF (Paraformaldéhyde) puis prélevés. Les tissus ont été congelés dans de l'OCT et des coupes de 5µm d'épaisseur ont été effectuées au niveau des valves aortiques. Ces coupes ont été ensuite colorées par de l'ORO (oil red O), spécifique des dépôts lipidiques, afin de mettre en évidence les lésions athéromateuses.

D'autres coupes de 5µm fixée dans du PFA ont été immunomarquées par des anticorps spécifiques du Nlrp3 (Santa Cruz Biotechnologies, sc-34408), de l'ASC (Santa Cruz Biotechnologies, sc-22514), du fragment p10 de la caspase-1 (Santa Cruz Biotechnologies, sc-514), de IL-1β clivée (Santa Cruz Biotechnologies, sc-23460) et de F4/80 (CALTAG Laboratories, MF48000). Des anticorps secondaires marqués à l'Alexa Fluor 488- (Molecular Probes) ou à la Cyanine-3 (Sigma) ont été utilisés pour révéler le marquage primaire. Des coupes marquées avec l'anticorps secondaire seul ou par une IgG de chèvre non spécifique ont été utilisées comme témoins négatifs.

Les coupes ont ensuite été colorées par du DAPI, montées et analysées par microscopie à fluorescence.

### Cytométrie de flux :

Après prélèvement sanguin, les érythrocytes ont été lysés puis les cellules restantes ont été marquées suivant le protocole classique. Les anticorps CD11b-biotin (clone M1/70) (BD Biosciences, révélés par de la streptavidine-Alexa-405 (Molecular Probes)), Gr1-PerCP-Cy5.5 (BD Biosciences), F4/80-APC (e-Bioscience) ont été utilisés pour le marquage membranaire.

L'analyse de l'expression de Nlrp3 et du fragment p10 de la caspase-1 par les monocytes a été effectuée sur le sang périphérique de souris LDLR-/- nourries pendant six semaines avec un régime riche en cholestérol. Les monocytes GR1high ou GR1low ont été incubés avec les anticorps primaires (ou avec les isotypes non spécifiques pour le contrôle négatif) puis avec l'anticorps secondaire conjugué à la cyanine 3. Les échantillons ont été finalement analysés par cytométrie de flux.

### Analyse de l'expression par RT-PCR

L'ARN a été extrait en utilisant le Rneasy mini-kit (Qiagen) suivant les instructions du fabricant. Un µg d'ARN a été soumis à une réaction de RT-PCR (iScript Reverse Transcriptase (BioRad)). L'ADN complémentaire généré a été ensuite amplifié en utilisant les oligonucléotides suivant
Nlrp3 :
   5'-gcaggaggaagactttgtgc-3'
   5'- aggagatgtcgaagcagcat-3'
Caspase-1:
   5'-accctcaagttttgcccttt-3',
   5'-tctttcaagcttgggcactt-3'
IL-1β:
   5'-ctcacaagcagagcacaagc-3'
   5'-ctcagtgcaggctatgacca-3'
IL-18 :
   5'-gcctcaaaccttccaaatca-3'
   5'-tggatccatttcctcaaagg-3'
L19 :
   5'-CTGAAGGTCAAAGGGAATGTG-3'
   5'-GGACAGAGTCTTGATGATCTC-3'.

L'ADN obtenu a été analysé par densitométrie et sa quantité a été estimée grâce au logiciel QuantityOne (Biorad) après normalisation par rapport au signal obtenu après amplification avec les oligonucléotides spécifiques du L19.

### Analyse statistique

Les résultats obtenus ont été considérés comme significatifs après analyse par le test de Student pour une valeur de p<0.05.

### Résultats :

Les inflammasomes Nlrp3 sont présents dans les lésions athéromateuses des souris LDLR -/- soumises à un régime riche en cholestérol.

L'expression des composants des inflammasomes Nlrp3 a été mise en évidence dans les lésions athéromateuses de l'aorte de souris LDLR-/- soumises à un régime de 3, 6 ou 10 semaines riche en cholestérol.

L'immunomarquage des souris soumises à un régime riche en cholestérol montre que les lésions athéromateuses expriment le Nlrp3, l'ASC, la sous unité active p10 de la caspase-1 et la sous unité active p17 de l'IL-1β. Cette expression est observée dès 3 semaines après le début du régime riche en cholestérol et est toujours présente après 10 semaines de celui ci. La superposition des différents immunomarquages a permis de mettre en évidence une colocalisation de l'expression des différents composants de l'inflamasomme Nlrp3. Cette expression est également détectée dans les régions comprenant des macrophages F4/80.

L'expression de Nlrp3 et de la caspase-1 a été mesurée par RT PCR sur des macrophages dérivés de la population de monocytes Gr1^{high} et Gr1^{low} obtenus par recrutement péritonéal. Après stimulation de ces cellules par des oxLDL, l'expression de Nlrp3 est significativement augmentée dans les cellules traitées par rapport aux cellules contrôles traitées aux LDL natives. Par contre, aucune différence significative n'a pu être observée pour l'expression de la caspase-1.

Nlrp3 et le fragment p10 de la caspase-1 sont surexprimées par les monocytes circulants des souris soumises à un régime riche en cholestérol. L'expression de Nlrp3 et du fragment p10 de la caspase-1 a été mesurée par cytométrie de flux chez les monocytes Gr1^{high} et Gr1^{low} des souris soumises à un régime riche en cholestérol. Cette expression est significativement plus importante chez les monocytes Gr1^{high} des souris soumises à un régime riche en cholestérol que chez les mêmes monocytes des souris soumises à un régime normal. Par contre, aucune différence significative n'a pu être observée dans l'expression de Nlrp3 et du fragment p10 de la caspase-1 dans les monocytes Gr1^{low}.

## Revendications

1. Procédé de diagnostic in vitro de la maladie athéromateuse à partir du sang d'un patient ou d'un animal **caractérisé en ce qu'**il comprend la mesure du niveau d'expression de Nlrp3, de l'ASC et/ou de caspase-1 par les cellules GR1high.

2. Procédé de diagnostic in vitro de la maladie athéromateuse selon la revendication 1 **caractérisé en ce que** ladite mesure du niveau d'expression de Nlrp3, de l'ASC et/ou de caspase-1 est la mesure du niveau d'expression de l'ARNm codant Nlrp3 ou caspase-1.

3. Procédé de diagnostic in vitro de la maladie athéromateuse selon l'une des revendications 1 à 2 **caractérisé en ce que** ladite mesure du niveau d'expression de l'ARNm codant Nlrp3, l'ASC ou la caspase-1 est faite par RT-PCR quantitative.

4. Procédé de diagnostic in vitro de la maladie athéromateuse selon l'une des revendications 1 à 3 **caractérisé en ce que** ladite mesure du niveau d'expression de Nlrp3, de l'ASC et/ou de caspase-1 est la mesure de la quantité de la protéine Nlrp3, de l'ASC et/ou de la protéine caspase-1 à l'intérieur des cellules GR1high.

5. Procédé de diagnostic in vitro de la maladie athéromateuse selon l'une des revendications 1 à 4 **caractérisé en ce que** ladite mesure de la quantité de la protéine Nlrp3, de l'ASC et/ou de la protéine caspase-1 à l'intérieur des cellules GR1high est faite par une méthode immunologique.

6. Procédé de diagnostic in vitro de la maladie athéromateuse selon l'une des revendications 1 à 5 **caractérisé en ce qu'**il comprend en outre une étape ou la protéine Nlrp3, l'ASC et/ou de caspase-1 est mise en contact avec un anticorps spécifique de ladite protéine.

7. Procédé de diagnostic in vitro de la maladie athéromateuse selon l'une des revendications 1 à 6 **caractérisé en ce que** ladite méthode immunologique est choisie parmi le groupe comprenant l'ELISA, la Cytométrie de flux et le Western-blot.

8. Procédé de diagnostic in vitro de la maladie athéromateuse selon l'une des revendications 1 à 7 **caractérisé en ce qu'**il comprend en outre une étape permettant d'augmenter la proportion des cellules GR1high par rapport aux autres cellules présentes dans le sang.

## Patentansprüche

1. Verfahren zur in vitro-Diagnose von atheromatöser Krankheit ausgehend vom Blut eines Patienten oder eines Tiers, **dadurch gekennzeichnet, dass** es die Messung des Expressionsniveaus von N1rp3, von ASC und/oder von Caspase-1 durch die GR1high-Zellen umfasst.

2. Verfahren zur in vitro-Diagnose von atheromatöser Krankheit nach Anspruch 1, **dadurch gekennzeichnet, dass** die Messung des Expressionsniveaus von N1rp3, von ASC und/oder von Caspase-1die Messung des Expressionsniveaus von ARNm, codierend N1rp3 oder Caspase-1, ist.

3. Verfahren zur in vitro-Diagnose von atheromatöser Krankheit nach einem der Ansprüche 1 bis 2, **dadurch gekennzeichnet, dass** die Messung des Expressionsniveaus von ARN, codierend N1rp3, ASC oder Caspase-1, durch quantitative RT-PCR erfolgt.

4. Verfahren zur in vitro-Diagnose von atheromatöser Krankheit nach einem der Ansprüche 1 bis 3, **dadurch gekennzeichnet, dass** die Messung des Expressionsniveaus von N1rp3, von ASC und/oder von Caspase-ldie Messung der Menge des Proteins N1rp3, von ASC und/oder des Proteins Caspase-1 im Inneren der GR1high-Zellen ist.

5. Verfahren zur in vitro-Diagnose von atheromatöser Krankheit nach einem der Ansprüche 1 bis 4, **dadurch gekennzeichnet, dass** die Messung der Menge des Proteins N1rp3, von ASC und/oder des Proteins Caspase-1 im Inneren der GR1high-Zellen durch ein immunologisches Verfahren erfolgt.

6. Verfahren zur in vitro-Diagnose von atheromatöser Krankheit nach einem der Ansprüche 1 bis 5, **dadurch gekennzeichnet, dass** es außerdem einen Schritt umfasst, in dem das Protein N1rp3, ASC und/oder Caspase-1 mit einem spezifischen Antiköper des Proteins in Kontakt gebracht wird.

7. Verfahren zur in vitro-Diagnose von atheromatöser Krankheit nach einem der Ansprüche 1 bis 6, **dadurch gekennzeichnet, dass** das immunologische Verfahren ausgewählt ist aus der Gruppe umfassend ELISA, Durchflusszytometrie und Western Blot.

8. Verfahren zur in vitro-Diagnose von atheromatöser Krankheit nach einem der Ansprüche 1 bis 7, **dadurch gekennzeichnet, dass** es außerdem einen Schritt umfasst, der ermöglicht, die Proportion der Zellen GR1high mit Bezug auf die anderen Zellen, die im Blut vorhanden sind, zu erhöhen.

## Claims

1. Method for the in vitro diagnosis of atheromatous disease using blood from a patient or an animal **characterised in that** it comprises measuring the expression level of N1rp3, ASC and/or caspase-1 by GR1high cells.

2. Method for the in vitro diagnosis of atheromatous disease according to claim 1, **characterised in that** said measurement of the expression level of Nlrp3, ASC and/or caspase-1 is the measurement of the expression level of the mRNA encoding Nlrp3 or caspase-1.

3. Method for the in vitro diagnosis of atheromatous disease according to any of claims 1 to 2, **characterised in that** said measurement of the expression level of the mRNA encoding Nlrp3 or caspase-1 is performed by means of quantitative RT-PCR.

4. Method for the in vitro diagnosis of atheromatous disease according to any of claims 1 to 3, **characterised in that** said measurement of the expression level of Nlrp3, ASC and/or caspase-1 is the measurement of the quantity of Nlrp3 protein, ASC and/or caspase-1 protein inside GR1high cells.

5. Method for the in vitro diagnosis of atheromatous disease according to any of claims 1 to 4, **characterised in that** said measurement of the quantity of N1rp3 protein, ASC and/or caspase-1 protein inside GR1high cells is performed by means of an immunological method.

6. Method for the in vitro diagnosis of atheromatous disease according to any of claims 1 to 5, **characterised in that** it further comprises a step wherein Nlrp3 protein, ASC and/or caspase-1 protein is placed in contact with a specific antibody for said protein.

7. Method for the in vitro diagnosis of atheromatous disease according to any of claims 1 to 6, **characterised in that** said immunological method is chosen from the group comprising ELISA, flow cytometry and Western Blot.

8. Method for the in vitro diagnosis of atheromatous disease according to any of claims 1 to 7, **characterised in that** it further comprises a step for increasing the proportion of GR1high cells in relation to the other cells contained in the blood.
